(19) Europäisches Patentamt
European Patent Office
Office européen des brevets

(11) Veröffentlichungsnummer: **0 052 798**
**B1**

(12) # EUROPÄISCHE PATENTSCHRIFT

(45) Veröffentlichungstag der Patentschrift: **08.05.85**

(51) Int. Cl.⁴: **C 07 D 215/22**, C 07 D 241/44, C 07 D 213/64, A 01 N 43/42, A 01 N 43/60, A 01 N 43/40

(21) Anmeldenummer: **81109201.4**

(22) Anmeldetag: **29.10.81**

(54) **Oximester, Verfahren zu deren Herstellung, ihre Verwendung, sowie diese Oximester enthaltende Mittel.**

(30) Priorität: **26.11.80 CH 8750/80**
**01.10.81 CH 6328/81**

(43) Veröffentlichungstag der Anmeldung:
**02.06.82 Patentblatt 82/22**

(45) Bekanntmachung des Hinweises auf die Patenterteilung:
**08.05.85 Patentblatt 85/19**

(84) Benannte Vertragsstaaten:
**AT BE CH DE FR IT LI NL SE**

(56) Entgegenhaltungen:
**EP - A - 0 001 473**
**EP - A - 0 003 114**
**EP - A - 0 023 785**
**EP - A - 0 029 319**
**EP - A - 0 050 097**
**DE - A - 2 262 402**
**DE - A - 3 004 770**
**US - A - 3 771 995**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Fráter, Georg, Dr., Am Pfisterhölzli 22, CH-8606 Greifensee (CH)**
Erfinder: **Suchy, Milos, Dr., Grossplatzstrasse 3, CH-8122 Pfaffhausen (CH)**
Erfinder: **Wenger, Jean, Dr., Brunnenwiesenstrasse 1, CH-8160 Uster (CH)**
Erfinder: **Winternitz, Paul, Dr., Am Pfisterhölzli 50, CH-8606 Greifensee (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

EP 0 052 798 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft Verbindungen der allgemeinen Formel

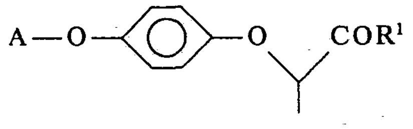
(I)

worin A eine der Gruppen

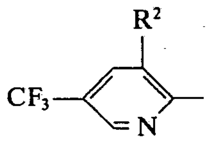
und
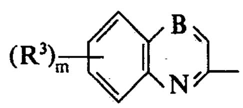

(Ia) (Ib)

darstellt, in welchen $R^2$ Wasserstoff, Halogen oder Trifluormethyl und $R^3$ Wasserstoff, Halogen oder Trifluormethyl, m 1 oder 2, B $-CH=$ oder Stickstoff bedeutet, und worin $R^1$ die Gruppe

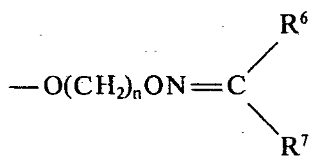
(Id)

darstellt, worin $R^6$ und $R^7$ $C_1-C_6$-Alkyl, Cycloalkyl mit 3 bis 6 Kohlenstoffatomen, $C_1-C_6$-Alkylcarbonyloxy-$C_1-C_6$-alkyl, $C_1-C_6$-Alkoxy, $C_1-C_6$-Alkylthio, oder zusammen mit dem Kohlenstoffatom, an das sie geknüpft sind, einen Cycloalkylrest mit 4 bis 7 Kohlenstoffatomen bedeuten, worin n 1 oder 2 bedeutet, und worin $R^6$ auch Wasserstoff bedeuten kann.

Die Erfindung betrifft ferner ein Verfahren zur Herstellung von Verbindungen der Formel I, Vorauflauf- und Nachauflauf-herbicide Mittel, Verfahren zur Herstellung solcher Mittel und zu deren Verwendung, sowie die Verwendung derartiger herbicider Mittel an sich.

Das Verfahren zur Herstellung von Verbindungen der Formel I ist dadurch gekennzeichnet, daß man eine Verbindung der allgemeinen Formel

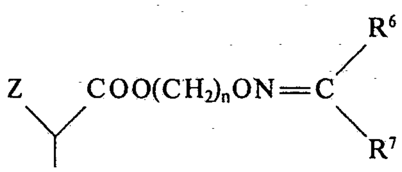
(V)

worin Z eine Abgangsgruppe bedeutet und $R^6$ und $R^7$ die oben angegebene Bedeutung besitzen, mit einer Verbindung der allgemeinen Formel

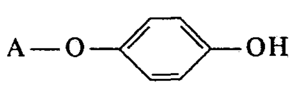
(VI)

worin A die oben angegebene Bedeutung besitzt, oder einem Alkalimetallsalz davon, erforderlichenfalls in Gegewart einer Base, umsetzt.

Der Ausdruck »$C_1-C_6$-Alkyl« umfaßt — wenn nicht anderweitig angegeben — sowohl geradkettige als auch verzweigte Kohlenwasserstoffradikale mit 1—6 Kohlenstoffatomen, wie Methyl, Äthyl, Propyl, Isopropyl, Butyl, Isobutyl, tert.-Butyl u. dgl. Alkylgruppen mit 1—3 Kohlenstoffatomen sind bevorzugt.

Unter »Halogen« sind Fluor, Chlor, Brom und Jod zu verstehen, wobei Chlor und Jod bevorzugt sind.

Von den Cycloalkylen mit 3—6 bzw. 4—7 Kohlenstoffatomen, nämlich Cyclopropyl, Cyclobutyl, Cyclopentyl, Cyclohexyl und Cycloheptyl ist das Cyclohexyl bevorzugt.

In den $C_1-C_6$-Alkoxy- bzw. $C_1-C_6$-Alkylthiogruppen enthalten die Alkylanteile 1—6, vorzugsweise 1—3 Kohlenstoffatome.

Bevorzugte Verbindungen der Formel I sind solche, worin $R^6$ und $R^7$ Alkylgruppen mit 1 oder 2 Kohlenstoffatomen, insbesondere Methyl, darstellen.

Das Symbol Z (Formel V) als Abgangsgruppe bedeutet insbesondere Chlor, Brom, Jod, Mesyloxy und Tosyloxy, sowie eine aktivierte Hydroxygruppe, insbesondere eine durch Umsetzung mit Triphenylphosphin und Azodicarbonsäure oder einem Ester davon, insbesondere Azodicarbonsäurediäthylester, aktivierte Hydroxygruppe (siehe z. B. Bull. Chem. Soc. Japan 46, 2833 (1973) oder Angew. Chem. 88, 111 (1976)).

Aufgrund des asymmetrischen Kohlenstoffatoms zwischen dem Sauerstoff und der Gruppe $-COR^1$ in den Verbindungen der Formel I können diese Verbindungen als Racemate oder in Form der optischen Isomeren vorliegen. Die D-Form der Verbindungen der Formel I ist im Hinblick auf ihre besonderen Wirkungsqualitäten bevorzugt.

Besonders bevorzugte Verbindungen der Formel I sind:

[(Isopropyliden-amino)oxy]methyl-2-[p-[(fluor-2-chinolyl]oxy]phenoxy]propionat und
[Isopropyliden-amino)oxy]methyl-2-[p-[(6-chlor-2-chinoxalinyl)oxy]phenoxy]propionat,
insbesondere die D-Isomeren dieser Verbindungen.

Aus den europäischen Patentanmeldungen EP-A1-50097, EP-A-3114 und EP-A-1473 und der D-OS 3 004 770 sind herbizid wirksame Verbindungen bekanntgeworden,von welchen sich die erfindungsgemäßen Verbindungen im wesentlichen durch die charakteristische Gruppierung Id unterscheiden.

Nach dem erfindungsgemäßen Verfahren zur Herstellung von Verbindungen der Formel I wird eine Verbindung der Formel V mit einer Verbindung der Formel VI oder einem Alkalimetallsalz davon in an sich bekannter Weise umgesetzt, erforderlichenfalls in Gegenwart einer Base. Die Umsetzung erfolgt zweckmäßigerweise in einem inerten organischen Lösungsmittel wie Kohlenwasserstoffen, z. B. Benzol oder Toluol, Äthern, z. B. Diäthyläther, Tetrahydrofuran, Dimethoxyäthan oder Hexamethylphosphorsäuretriamid, u. dgl. Temperatur und Druck sind nicht kritisch und man arbeitet bevorzugt bei einer Temperatur zwischen −20° und der Rückflußtemperatur der Reaktionsmischung, vorzugsweise zwischen −10° und 30° C.

Die Verbindungen der allgemeinen Formel I schließen, wie oben ausgeführt, auch optische Isomere ein, da sie ein asymmetrisches Kohlenstoffatom in der $\alpha$-Stellung besitzen. Weitere asymmetrische Kohlenstoffatome können die Esterkomponenten enthalten. Erwünschtenfalls können die racemischen Verbindungen unter Verwendung bekannter Verfahren in rechtsdrehende und linksdrehende Verbindungen getrennt werden. Derartige Verfahren sind beispielsweise in Industrial and Engineering Chemistry 60 (8) 12—28 beschrieben. Die Isomeren und die racemischen Mischungen besitzen alle herbicide Aktivität, jedoch ist das Ausmaß dieser Aktivität unterschiedlich. Am größten ist die Aktivität beim D-Isomeren, danach folgt die racemische Mischung und dann das L-Isomere.

Die Isomeren — insbesondere die D-Isomeren — können auch durch Synthese aus entsprechenden optisch aktiven Ausgangsmaterialien hergestellt werden.

Infolge der Stickstoff-Kohlenstoff-Doppelbindung in der

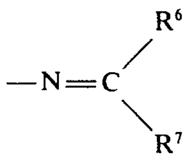

Gruppe erhält man jeweils zwei geometrische Isomere (wenn $R^6$ und $R^7$ unterschiedliche Bedeutung haben), die als syn- und anti-Form bezeichnet werden. Es gelingt in gewissen Fällen, derartige Isomere zu isolieren. Diese sind ebenfalls Gegenstand der Erfindung.

Die vorliegende Erfindung betrifft auch herbicide Zusammensetzungen, die als aktiven Bestandteil ein oder mehrere Verbindungen der Form I, wie oben definiert, enthalten. Die herbicide Zusammensetzung enthält zweckmäßigerweise zumindest eines der folgenden Materialien: Trägerstoffe, Netzmittel, inerte Verdünnungsmittel und Lösungsmittel.

Die erfindungsgemäßen Vorauflauf- und Nachauflauf-Herbicide eignen sich besonders zur Bekämpfung von Ungräsern, insbesondere von Ackerfuchsschwanz (Alopecurus myosuroides) und Hirsearten wie beispielsweise Hühnerhirse (Echinochloa crus-galli), große Borstenhirse (Setaria faberii) und haarartige Hirse (Panicum capillare) in Getreide — insbesondere Gerste-, Hafer- und Weizen- sowie Reis-, Baumwolle-, Soya-Zuckerrüben und Gemüsekulturen. Besonders bevorzugt eignen sich die erfindungsgemäßen Vorauflauf- und Nachauflauf-Herbicide zur Bekämpfung von Ungräsern in Zuckerrübenkulturen. Im allgemeinen genügt eine Konzentration von 0,1—6 kg/ha, vorzugsweise 0,6—2,0 kg/ha, besonders bevorzugt 1—1,5 kg/ha, um den gewünschten herbiciden Effekt mit Verbindungen der Formel I zu erzielen. Die erfindungsgemäßen Verbindungen sind gegen Alopecurus besonders wirksam.

Für den Fall, daß $R^3$ den Trifluormethylrest darstellt, können diese Verbindungen nur bedingt im Getreidebau Verwendung finden, da etwas Phytotoxizität eintritt. Diese Verbindungen eignen sich jedoch besonders zur Bekämpfung von Ungräsern in Reis-, Baumwoll-, Soya-, Zuckerrüben- und Gemüsekulturen.

Die Verbindungen der Formel I sind im allgemeinen wasserunlöslich und können nach irgendeiner der für unlösliche Verbindungen üblichen Methoden konfektioniert werden.

Wenn gewünscht, können die Verbindungen der Formel I in einem mit Wasser nicht mischbaren Lösungsmittel, wie beispielsweise einem hochsiedenden Kohlenwasserstoff, gelöst werden, das zweckmäßigerweise gelöste Emulgatoren enthält, so daß es bei Zugabe zu Wasser als selbstemulgierbares Öl wirkt.

Die Verbindungen der Formel I können auch mit einem Netzmittel mit oder ohne inertes Verdünnungsmittel zur Bildung eines netzbaren Pulvers vermischt werden, welches in Wasser löslich oder dispergierbar ist, oder sie können mit dem inerten Verdünnungsmittel zur Bildung eines festen oder pulverförmigen Produktes vermischt werden.

Inerte Verdünnungsmittel, mit welchen die Verbindungen der Form I verarbeitet werden können, sind feste inerte Medien, einschließlich pulverförmige oder feinverteilte Feststoffe, wie beispielsweise Tone, Sande, Talk, Glimmer, Düngemittel u. dgl., wobei solche Produkte entweder staubförmig oder als Materialien mit größerer Teilchengröße vorliegen können.

Die Netzmittel können anionische Verbindungen, wie beispielsweise Seifen, Fettsulfatester, wie Dodecylnatriumsulfat, Octadecylnatriumsulfat und Cetylnatriumsulfat, fettaromatische Sulfonate, wie Alkylbenzolsulfonate oder Butylnaphthalinsulfonate, komplexere Fettsulfonate, wie die Amidkondensationsprodukte von Ölsäure und N-Methyltaurin oder das Natriumsulfonat von Dioctylsuccinat sein.

Die Netzmittel können auch nichtionische Netzmittel, wie beispielsweise Kondensationsprodukte von Fettsäuren, Fettalkoholen oder fettsubstituierten Phenolen mit Äthylenoxyd, oder Fettsäureester und -äther von Zuckern oder mehrwertigen Alkoholen oder die Produkte, die aus letzteren durch Kondensation mit Äthylenoxyd erhalten werden, oder die Produkte, die als Blockcopolymere von Äthylenoxyd und Propylenoxyd bekannt sind, sein. Die Netzmittel können auch kationische Mittel, wie beispielsweise Cetyltrimethylammoniumbromid u. dgl., sein.

Die herbicide Zusammensetzung kann auch in Form einer Aerosolverbindung vorliegen, wobei zweckmäßigerweise zusätzlich zum Treibgas, welches geeigneterweise ein polyhalogeniertes Alkan, wie Dichlordifluormethan ist, ein Co-Solvens und ein Netzmittel verwendet wird.

Die herbiciden Zusammensetzungen, gemäß der vorliegenden Erfindung können zusätzlich zu den Verbindungen der Formel I, Synergisten und andere aktive Insektizide, Bakterizide, Herbicide und Fungizide enthalten.

In ihren verschiedenen Anwendungsgebieten können die erfindungsgemäßen Verbindungen in unterschiedlichen Mengenverhältnissen eingesetzt werden.

Die erfindungsgemäßen Unkrautbekämpfungsmittel können in einer Form vorliegen, die sich für die Lagerung und den Transport eignen. Solche Formen können z. B. 2—90% an einem oder mehreren Wirkstoffen der Formel I enthalten. Diese Formen können dann mit gleichen oder verschiedenen Trägermaterialien bis zu Konzentrationen verdünnt werden, die sich für den praktischen Gebrauch eignen. Im gebrauchsfertigen Mittel können dann Wirkstoffkonzentrationen von 2—80% (Gewichtsprozent) vorliegen. Die Wirkstoffkonzentration kann jedoch auch kleiner oder größer sein. Je nach Verwendungszweck ist eine Wirkstoffkonzentration von 2—8% bzw. 50—80% besonders bevorzugt.

Die folgenden Beispiele sollen die vorliegende Erfindung illustrieren. Alle Temperaturen sind in °C angegeben.

## Beispiel 1

Durch Reaktion von D-2-[p-[[5-(Trifluormethyl)-2-pyridyl]oxy]phenoxy]propionsäure mit Isopropylidenaminooxyäthanol erhält man das 2-[(Isopropylidenamino)oxy]äthyl-D-2-[p-[[5-(trifluormethyl)-2-pyridyl]oxy]phenoxy]propionat. Diese Reaktion erfolgt in Analogie zu folgendem Referenzbeispiel:

3,4 g 2-[p-[[(5-Trifluormethyl)-2-pyridyl]oxy]phenoxy]-propionsäure werden in 50 ml Dichlormethan suspendiert und bei Raumtemperatur werden 1,1 g Acetonoxim zugegeben. Danach werden 2,1 g Dicyclohexylcarbodiimid, gelöst in 20 ml Dichlormethan, über einen Zeitraum von 10 Minuten, zugetropft; dabei steigt die Temperatur bis gegen 40°C an. Es wird 2 Stunden bei Raumtemperatur nachgerührt. Das Reaktionsgemisch wird filtriert und am Rotationsverdampfer zur Trockene eingedampft. Man erhält so Aceton O-[2-[p-[[5-(Trifluormethyl)-2-pyridyl]oxy]phenoxy]propionyl]oxim. $n_D^{24}$ 1,5202.

Die oben als Ausgangsmaterial verwendete 2-[p-[[[5-(Trifluormethyl)-2-pyridyl]oxy]propionsäure kann wie folgt erhalten werden:

0,7 g einer 50%igen Suspension von Natriumhydrid in Mineralöl werden in einer Inertgasatmosphäre zweimal mit je 5,0 ml Tetrahydrofuran gewaschen, dann in 15,0 ml Tetrahydrofuran eingetragen und tropfenweise mit einer Lösung von 3,5 g p-(5-[Trifluormethyl-2-pyridyl)oxy]phenol, gelöst in 30,0 ml Dimethylformamid, versetzt. In das Gemisch werden anschließend 3,5 g L-2-[(p-Tolylsulfonyl)oxy]propionsäureäthylester in 20,0 ml Dimethylformamid eingetropft. Das Reaktionsgemisch wird 12 Stunden bei Raumtemperatur nachgerührt, danach auf Eis gegossen und erschöpfend mit Äther extrahiert. Der Ätherextrakt wird mit Wasser gewaschen, über Natriumsulfat getrocknet und unter vermindertem Druck eingedampft. Der zurückbleibende D-2-[p-[[5-[Trifluormethyl)-2-pyridyl]oxy]phenoxy]propionsäureäthylester kann durch Adsorption an Kieselgel gereinigt werden; $[\alpha]_D^{20}$ +21,7° ($CHCl_3$; c=1,1%)

5,3 g D-2-[p-[[5-(Trifluormethyl)-2-pyridyl]oxy]phenoxy]propionsäureäthylester werden in 20 ml Methanol suspendiert, mit 1,7 g KOH, gelöst in 50 ml Wasser, versetzt und während 1$^1/_2$ Stunden bei Raumtemperatur gerührt. Zur Aufarbeitung wird das Gemisch mit 200 ml Wasser verdünnt, mit HCl angesäuert und mit Äther extrahiert. Der Ätherextrakt wird mit Wasser gewaschen, über Natriumsulfat getrocknet und unter vermindertem Druck eingedampft. Die zurückbleibende D-2-[p-[[5-(Trifluormethyl)-2-pyridyl]oxy]phenoxy]propionsäure kann durch Adsorption an Kieselgel gereinigt werden; $[\alpha]_D^{22}$ = +18,3° ($CHCl_3$, c = 1,1%).

In analoger Weise erhält man beim Einsatz von p-[(3-Chlor-5-trifluormethyl-2-pyridyl)oxy]phenol und L-2-[(p-Tolylsulfonyl)-oxy]propionsäureäthylester den D-2-[p-[(3-Chlor-5-trifluormethyl-2-pyridyl)oxy] phenoxy]propionsäureäthylester und daraus die D-2-[p-[(3-Chlor-5-trifluormethyl-2-pyridyl)-oxy]phenoxy] propionsäure sowie das entsprechende D-Isomere des Oximesters.

## Beispiel 2

1,75 g [(Isopropylidenamino)-oxy]methyl L(—)-lactat, 2,65 g Triphenylphosphin und 2,72 g p-[(6-Chlor-2-chinoxalyl)-oxy]phenol werden bei 0°C in 10 ml absolutem Tetrahydrofuran gelöst. Zur erhaltenen Lösung tropft man unter Rühren und Kühlung 1,75 g Azodicarbonsäurediäthylester. Hierauf rührt man eine halbe Stunde nach, gießt auf 100 ml Wasser und extrahiert 2mal mit Äthylacetat.

Die organische Phase wird mit verdünnter Salzsäure und Wasser nachgewaschen, über Natriumsulfat getrocknet und eingedampft.

Der Rückstand wird über die 20fache Menge Kieselgel chromatographiert. (Laufmittel Hexan zu Äthylacetat/8 : 2). Das eluierte Produkt wird aus Methylenchlorid/n-Hexan umkristallisiert. Man erhält [(Isopropylidenamino)-oxy]-methyl-D-2-[p-[(6-chlor-2-chinoxalyl)-oxy]phenoxy]-propionat, $[\alpha]_D^{20}$ = +20,05 (c = 1,93% in $CHCl_3$); FP: 75—77°C.

Analog erhält man aus einem 1 : 1 Gemisch von p-(6-Fluor-2-chinoxalyloxy)-phenol und p-(7-Fluor-2-chinoxalyloxy)-phenol ein 1 : 1 Gemisch von [(Isopropylidenamino)oxy]-methyl D-2-[p-[(6-fluor-2-chinoxalinyl)oxy]phenoxy]propionat und [(Isopropylidenamino)oxy]methyl D-2-[p-[(7-fluor-2-chinoxalinyl)oxy]phenoxy]propionat, $[\alpha]_D^{22}$ = +21,18 (c = 1,14% in $CHCl_3$).

## Beispiel 3

Zur Herstellung eines emulgierbaren Konzentrates werden die nachstehend aufgeführten Bestandteile dieses Konzentrates miteinander vermischt:

| | |
|---|---|
| Verbindung der Formel I | 500 g |
| Kondensationsprodukte eines Alkylphenols und Äthylenoxyd; | |
| Dodecylbenzolsulfonsaures Calcium | 100 g |
| Epoxydiertes Soyaöl mit einem Oxiransauerstoffgehalt von ca. 6% | 25 g |
| Butyliertes Hydroxytoluol | 10 g |

Diese Mischung wird mit Xylol auf 1 Liter aufgefüllt.

**Patentansprüche für die Vertragsstaaten BE, CH, DE, FR, IT, LI, NL, SE**

1. Heterocyclische Verbindungen der allgemeinen Formel

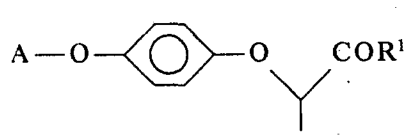

(I)

worin A eine der Gruppen

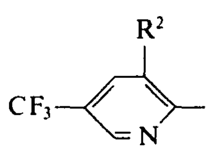

und

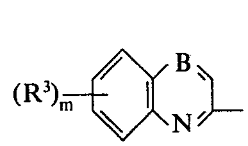

(Ia) (Ib)

darstellt, in welchen $R^2$ Wasserstoff, Halogen oder Trifluormethyl und $R^3$ Wasserstoff, Halogen oder Trifluormethyl, m 1 oder 2, B —CH= oder Stickstoff bedeutet, und worin $R^1$ die Gruppe

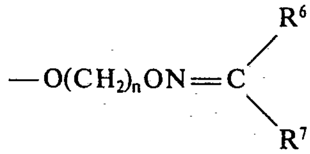

(Id)

darstellt, worin $R^6$ und $R^7$ $C_1-C_6$-Alkyl, Cycloalkyl mit 3 bis 6 Kohlenstoffatomen, $C_1-C_6$-Alkylcarbonyloxy-$C_1-C_6$-alkyl, $C_1-C_6$-Alkoxy, $C_1-C_6$-Alkylthio, oder zusammen mit dem Kohlenstoffatom, an das sie geknüpft sind, einen Cycloalkylrest mit 4 bis 7 Kohlenstoffatomen bedeuten, worin n 1 oder 2 bedeutet, und worin $R^6$ auch Wasserstoff bedeuten kann.

2. Verbindungen nach Anspruch 1, dadurch gekennzeichnet, daß $R^2$ Halogen oder Trifluormethyl bedeutet.

3. Verbindungen nach Anspruch 1 oder 2, dadurch gekennzeichnet, daß A die Gruppe Ib bedeutet, worin B Stickstoff darstellt.

4. Verbindungen nach einem der Ansprüche 1—3, dadurch gekennzeichnet, daß $R^6$ und $R^7$ Alkylgruppen mit 1 oder 2 Kohlenstoffatomen, insbesondere Methyl, bedeuten.

5. [(Isopropylidenamino)oxy]methyl-2-[p-[(6-fluor-2-chinolyl)oxy]phenoxy]propionat.

6. [(Isopropylidenamino)oxy]methyl-2-[p-[(6-chlor-2-chinoxalinyl)oxy]phenoxy]propionat.

7. Die D-Isomeren der Verbindungen nach einem der Ansprüche 1—6.

8. Die Verbindungen nach einem der Ansprüche 1—7 als Herbizide.

9. Herbizides Mittel, dadurch gekennzeichnet, daß es eine Verbindung der allgemeinen Formel I in Anspruch 1 und inertes Trägermaterial enthält.

10- Herbizides Mittel, dadurch gekennzeichnet, daß es eine Verbindung nach einem der Ansprüche 2—7 und inertes Trägermaterial enthält.

11. Verfahren zur Herstellung von Verbindungen der allgemeinen Formel I in Anspruch 1, dadurch gekennzeichnet, daß man eine Verbindung der allgemeinen Formel

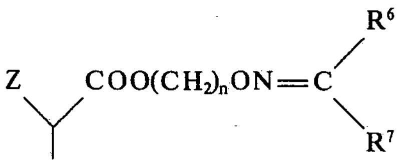

(V)

worin Z eine Abgangsgruppe bedeutet und $R^6$ und $R^7$ die oben angegebene Bedeutung besitzen, mit einer Verbindung der allgemeinen Formel

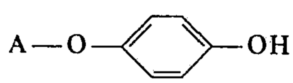

(VI)

worin A die oben angegebene Bedeutung besitzt, oder einem Alkalimetallsalz davon, erforderlichenfalls in Gegenwart einer Base, umsetzt.

12. Verfahren nach Anspruch 11, dadurch gekennzeichnet, daß man eine Verbindung der Formel V verwendet, worin Z Chlor, Brom, Jod, Mesyloxy oder Tosyloxy bedeutet.

13. Verfahren nach Anspruch 11 oder 12, dadurch gekennzeichnet, daß man als Ausgangsmaterialien der Formel V die D-Isomeren verwendet oder daß man aus einem erhaltenen Racemat das D-Isomere isoliert.

14. Verfahren nach Anspruch 11, 12 oder 13, dadurch gekennzeichnet, daß man als Ausgangsmaterial der Formel VI eine Verbindung verwendet, worin A die Gruppe Ib bedeutet, worin B Stickstoff darstellt.

15. Verfahren nach einem der Ansprüche 11—14, dadurch gekennzeichnet, daß man als Ausgangsmaterial eine Verbindung der Formel V verwendet, worin $R^6$ und $R^7$ Alkylgruppen mit 1 oder 2 Kohlenstoffatomen, insbesondere Methyl, bedeuten.

16. Verfahren zur Unkrautbekämpfung, dadurch gekennzeichnet, daß man das Unkraut mit einem Mittel gemäß Anspruch 9 oder 10 behandelt.

17. Verwendung der in den Ansprüchen 1—8 genannten Verbindungen als Unkrautbekämpfungsmittel.

**Patentansprüche für den Vertragsstaat AT**

1. Herbizides Mittel, dadurch gekennzeichnet, daß es eine Verbindung der allgemeinen Formel

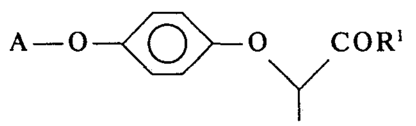

(I)

worin A eine der Gruppen

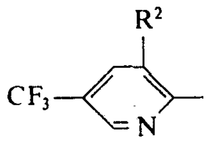

und

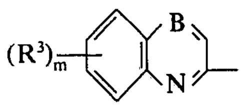

(Ia) (Ib)

darstellt, in welchen $R^2$ Wasserstoff, Halogen oder Trifluormethyl und $R^3$ Wasserstoff, Halogen oder Trifluormethyl, m 1 oder 2, B $-CH=$ oder Stickstoff bedeutet, und worin $R^1$ die Gruppe

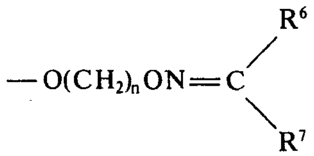

(Id)

darstellt, worin $R^6$ und $R^7$ $C_1-C_6$-Alkyl, Cycloalkyl mit 3 und 6 Kohlenstoffatomen, $C_1-C_6$-Alkyl-carbonyloxy-$C_1-C_6$-alkyl, $C_1-C_6$-Alkoxy, $C_1-C_6$-Alkylthio, oder zusammen mit dem Kohlenstoffatom, an das sie geknüpft sind, einen Cycloalkylrest mit 4 bis 7 Kohlenstoffatomen bedeuten, worin n 1 oder 2 bedeutet, und worin $R^6$ auch Wasserstoff bedeuten kann, und inertes Trägermaterial enthält.

2. Herbizides Mittel nach Anspruch 1, dadurch gekennzeichnet, daß es eine Verbindung der Formel I enthält, worin $R^2$ Halogen oder Trifluormethyl bedeutet.

3. Herbizides Mittel nach Anspruch 1 oder 2, dadurch gekennzeichnet, daß es eine Verbindung der Formel I enthält, in welcher A die Gruppe Ib bedeutet, worin B Stickstoff darstellt.

4. Herbizides Mittel nach einem der Ansprüche 1—3, dadurch gekennzeichnet, daß es eine Verbindung der Formel I enthält, worin die Substituenten $R^6$ und $R^7$ Alkylgruppen mit 1 oder 2 Kohlenstoffatomen, insbesondere Methyl, bedeuten.

5. Herbizides Mittel nach einem der Ansprüche 1—4, dadurch gekennzeichnet, daß es das D-Isomere einer Verbindung der Formel I enthält.

6. Herbizides Mittel nach einem der Ansprüche 1—5, dadurch gekennzeichnet, daß es [(Isopropylidenamino)oxy]methyl-2-[p-[(6-fluor-2-chinolyl)oxy]phenoxy]propionat oder [(Isopropylidenamino)oxy]methyl-2-[p-[(6-chlor-2-chinoxalinyl)oxy]phenoxy]propionat, insbesondere das D-Isomere einer dieser Verbindungen, enthält.

7. Verfahren zur Herstellung von Verbindungen der allgemeinen Formel I in Anspruch 1, dadurch gekennzeichnet, daß man eine Verbindung der allgemeinen Formel

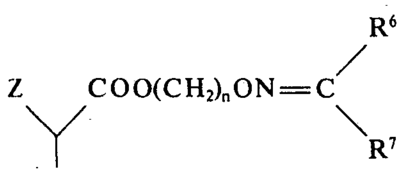

(V)

worin Z eine Abgangsgruppe bedeutet und $R^6$ und $R^7$ die oben angegebene Bedeutung besitzen, mit einer Verbindung der allgemeinen Formel

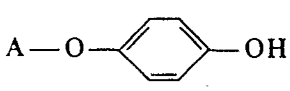

(VI)

worin A die oben angegebene Bedeutung besitzt, oder einem Alkalimetallsalz davon, erforderlichenfalls in Gegenwart einer Base, umsetzt.

8. Verfahren nach Anspruch 7, dadurch gekennzeichnet, daß man eine Verbindung der Formel V verwendet, worin Z Chlor, Brom, Jod, Mesyloxy oder Tosyloxy bedeutet.

9. Verfahren nach Anspruch 7 oder 8, dadurch gekennzeichnet, daß man als Ausgangsmaterialien

der Formel V die D-Isomeren verwendet oder daß man aus einem erhaltenen Racemat das D-Isomere isoliert.

10. Verfahren nach Anspruch 7, 8 oder 9, dadurch gekennzeichnet, daß man als Ausgangsmaterial der Formel VI eine Verbindung verwendet, worin A die Gruppe Ib bedeutet, worin B Stickstoff darstellt.

11. Verfahren nach einem der Ansprüche 7—10, dadurch gekennzeichnet, daß man als Ausgangsmaterial eine Verbindung der Formel V verwendet, worin $R^6$ und $R^7$ Alkylgruppen mit 1 oder 2 Kohlenstoffatomen, insbesondere Methyl, bedeuten.

12. Verfahren zur Herstellung von herbiziden Mitteln, dadurch gekennzeichnet, daß man eine der in den Ansprüchen 1—6 genannten Verbindungen unter Verwendung fester oder flüssiger Trägermaterialien und gegebenenfalls weiterer Hilfsmittel, in eine für seine Applikation geeignete Form bringt.

13. Verfahren zur Unkrautbekämpfung, dadurch gekennzeichnet, daß man das Unkraut mit einem Mittel gemäß einem der Ansprüche 1—6 behandelt.

14. Verwendung der in den Ansprüchen 1—6 genannten Verbindungen als Unkrautbekämpfungsmittel.

**Claims for the contracting states: BE, CH, DE, FR, IT, LI, NL, SE**

1. Heterocyclic compounds of the general formula

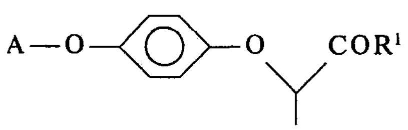 (I)

wherein A represents one of the groups

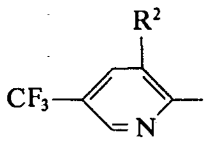 and 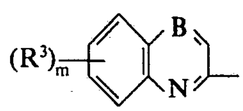

(Ia) (Ib)

in which $R^2$ signifies hydrogen, halogen or trifluoromethyl and $R^3$ signifies hydrogen, halogen or trifluoromethyl, m signifies 1 or 2, B signifies $-CH=$ or nitrogen, and wherein $R^1$ represents the group

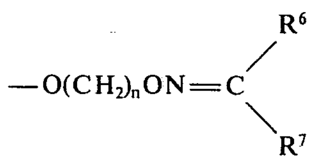 (Id)

wherein $R^6$ und $R^7$ signify $C_1-C_6$-alkyl, cycloalkyl with 3 to 6 carbon atoms, $C_1-C_6$-alkylcarbonyloxy-$C_1-C_6$-alkyl, $C_1-C_6$-alkoxy, $C_1-C_6$-alkylthio, or together with the carbon atom to which they are attached a cycloalkyl residue with 4 to 7 carbon atoms, wherein n signifies 1 or 2, and wherein $R^6$ can also signify hydrogen.

2. Compounds according to claim 1, characterized in that $R^2$ signifies halogen or trifluoromethyl.

3. Compounds according to claim 1 or 2, characterized in that A signifies the group Ib wherein B represents nitrogen.

4. Compounds according to any one of claims 1—3, characterized in that $R^6$ und $R^7$ signify alkyl groups with 1 or 2 carbon atoms, especially methyl.

5. [(Isopropylideneamino)oxy]methyl-2-[p-[(6-fluoro-2-quinolyl)oxy]phenoxy]propionate.

6. [(Isopropylideneamino)oxy]methyl-2-[p-[(6-chloro-2-quinoxalinyl)oxy]phenoxy]propionate.

7. The D-isomers of the compounds according to any one of claims 1—6.

8. The compounds according to any one of claims 1—7 as herbicides.

9. A herbicidal composition, characterized in that it contains a compound of general formula I in claim 1 and inert carrier material.

10. A herbicidal composition, characterized in that it contains a compound according to any one of claims 2—7 and inert carrier material.

11. A process for the manufacture of compounds of general formula I in claim 1, characterized by reacting a compound of the general formula

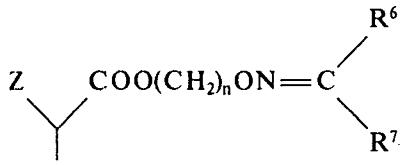

(V)

wherein Z signifies a leaving group and $R^6$ and $R^7$ have the significance given above, with a compound of the general formula

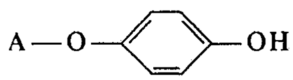

(VI)

wherein A has the significance given above, or an alkali metal salt thereof, if required in the presence of a base.

12. A process according to claim 11, characterized in that a compound of formula V wherein Z signifies chlorine, bromine, iodine, mesyloxy or tosyloxy is used.

13. A process according to claim 11 or 12, characterized in that the D-isomers are used as the starting materials of formula V or in that the D-isomer is isolated from a racemate obtained.

14. A process according to claim 11, 12 or 13, characterized in that a compound wherein A signifies the group Ib in which B represents nitrogen is used as the starting material of formula VI.

15. A process according to any one of claims 11—14, characterized in that a compound of formula V wherein $R^6$ and $R^7$ signify alkyl groups with 1 or 2 carbon atoms, especially methyl, is used as the starting material.

16. A method for the control of weeds, characterized by treating the weeds with a composition in accordance with claim 9 or 10.

17. The use of the compounds set forth in claims 1—8 as weed control agents.

**Claims for the contracting state: Austria**

1. A herbicidal composition, characterized in that it contains a compound of the general formula

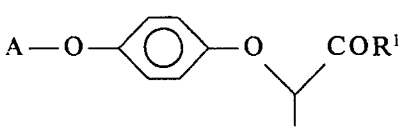

(I)

wherein A represents one of the groups

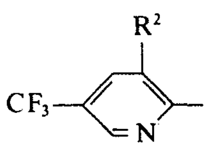

and

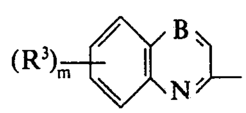

(Ia) (Ib)

in which $R^2$ signifies hydrogen, halogen or trifluoromethyl and $R^3$ signifies hydrogen, halogen or trifluoromethyl, m signifies 1 or 2, B signifies $-CH=$ or nitrogen, and wherein $R^1$ represents the group

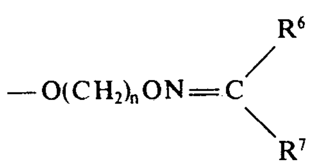

(Id)

wherein $R^6$ and $R^7$ signify $C_1-C_6$-alkyl, cycloalkyl with 3—6 carbon atoms, $C_1-C_6$-alkylcarbonyloxy-$C_1-C_6$-alkyl, $C_1-C_6$-alkoxy, $C_1-C_6$-alkylthio, or together with the carbon atom to which they are attached a cycloalkyl residue with 4 to 7 carbon atoms, wherein n signifies 1 or 2, and wherein $R^6$ can also signify hydrogen, and inert carrier material.

2. A herbicidal composition according to claim 1, characterized in that it contains a compound of formula I wherein $R^2$ signifies halogen or trifluoromethyl.

3. A herbicidal composition according to claim 1 or 2, characterized in that it contains a compound of formula I in which A signifies the group Ib wherein B represents nitrogen.

4. A herbicidal composition according to any one of claims 1—3, characterized in that it contains a compound of formula I wherein the substituents $R^6$ and $R^7$ signify alkyl groups with 1 or 2 carbon atoms, especially methyl.

5. A herbicidal composition according to any one of claims 1—4, characterized in that it contains the D-isomer of a compound of formula I.

6. A herbicidal composition according to any one of claims 1—5, characterized in that it contains [(isopropylideneamino)oxy]methyl 2-[p-[(6-fluoro-2-quinolyl)oxy]phenoxy]propionate or [(isopropyl ideneamino)oxy]methyl 2-[p-[(chloro-2-quinoxalinyl)oxy]phenoxy]propionate, especially the D-isomer of one of these compounds.

7. A process for the manufacture of compounds of general formula I in claim 1, characterized by reacting a compound of the general formula

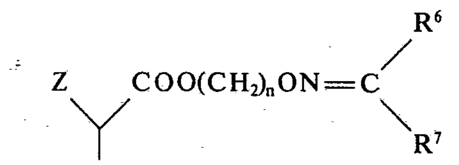

(V)

wherein Z signifies a leaving group and $R^6$ and $R^7$ have the significance given above, with a compound of the general formula

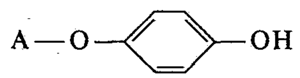

(VI)

wherein A has the significance given above, or an alkali metal salt thereof, if required in the presence of a base.

8. A process according to claim 7, characterized in that a compound of formula V wherein Z signifies chlorine, bromine, iodine, mesyloxy or tosyloxy is used.

9. A process according to claim 7 or 8, characterized in that the the D-isomers are used as the starting materials of formula V or in that the D-isomer is isolated from a racemate obtained.

10. A process according to claim 7, 8 or 9, characterized in that a compound wherein A signifies the group Ib in which B represents nitrogen is used as the starting material of formula VI.

11. A process according to any one of claims 7—10, characterized in that a compound of formula V wherein $R^6$ and $R^7$ signify alkyl groups with 1 or 2 carbon atoms, especially methyl, is used as the starting material.

12. A process for the manufacture of herbicidal compositions, characterized by bringing one of the compounds set forth in claims 1—6 into a form suitable for its application using solid or liquid carrier materials and optionally further adjuvants.

13. A method for the control of weeds, characterized by treating the weeds with a composition in accordance with any of claims 1—6.

14. The use of the compounds set forth in claims 1—6 as weed control agents.

**Revendications pour les Etats contractants: BE, CH, DE, FR, IT, LI, NL, SE**

1. Composés hétérocycliques de formule générale

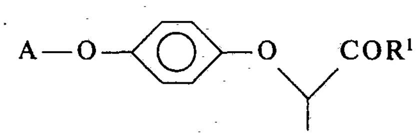

(I)

dans laquelle A représente l'un des groupes

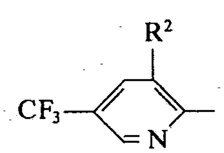

(Ia)

et

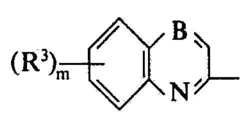

(Ib)

dans laquels $R^2$ représente l'hydrogène, un halogène ou le groupe trifluorométhyle et $R^3$ représente l'hydrogène, un halogène ou le groupe trifluorométhyle, m est égal à 1 ou 2, B représente $-CH=$ ou l'azote, et $R^1$ représente le groupe

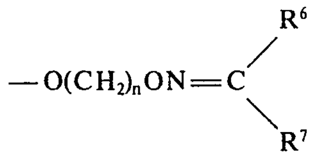

(Id)

dans lequel $R^6$ et $R^7$ représentent des groupes alkyle en C1—C6, cycloalkyle en C3—C6, (alkyle en C1—C6)-carbonyloxy-alkyle en C1—C6, alcoxy en C1—C6, alkylthio en C1—C6 ou bien forment ensemble et avec l'atome de carbone auquel ils sont reliés, un groupe cycloalkyle en C4—C7, n est égal à 1 ou 2, $R^6$ pouvant également représenter l'hydrogène.

2. Composés selon la revendication 1, caractérisés en ce que $R^2$ représente un halogène ou le groupe trifluorométhyle.

3. Composés selon la revendication 1 ou 2, caractérisés en ce que A représente le groupe Ib dans lequel B représente l'azote.

4. Composés selon l'une des revendications 1 à 3, caractérisés en ce que $R^6$ et $R^7$ représentent des groupes alkyle en C1 ou C2, en particulier des groupes méthyle.

5. Le 2-[p-[(6-fluoro-2-quinoléyl),oxy]phénoxy]-propionate d'[(isopropylidénamino)-oxy]-méthyle.

6. Le 2-[p-[(6-chloro-2-quinoxalinyl)-oxy]phénoxyl]-propionate d'[(isopropylidénamino)-oxy]-méthyle.

7. Les isomères D des composés selon l'une des revendications 1 à 6.

8. Les composés selon l'une des revendications 1 à 7 en tant qu'herbicides.

9. Produit herbicide caractérisé en ce qu'il contient un composé de formule générale I de la revendication 1 et un support inerte.

10. Produit herbicide caractérisé en ce qu'il contient un composé selon l'une des revendications 2 à 7 et un support inerte.

11. Procédé de préparation des composés de formule générale I de la revendication 1, caractérisé en ce que l'on fait réagir un composé de formule générale

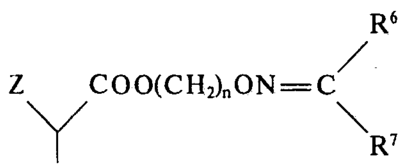

(V)

dans laquelle Z représente un groupe éliminable et $R^6$ et $R^7$ ont les significations indiquées ci-dessus, avec un composé de formule générale

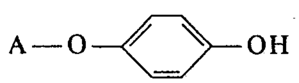

(VI)

dans laquelle A à la signification indiquée ci-dessus, ou un sel de métal alcalin d'un tel composé, en présence d'une base lorsque c'est nécessaire.

12. Procédé selon la revendication 11, caractérisé en ce que l'on utilise un composé de formule V dans laquelle Z représente le chlore, le brome, l'iode, un groupe méthane-sulfonyloxy ou toluène-sulfonyloxy.

13. Procédé selon la revendication 11 ou 12, caractérisé en ce que l'on utilise en tant que produits de départ de formule V les isomères D ou en ce que l'on isole l'isomère D d'un racémate obtenu.

14. Procédé selon la revendication 11, 12 ou 13, caractérisé en ce que l'on utilise en tant que produit de départ de formule VI un composé dans lequel A représente le groupe Ib dans lequel B représente l'azote.

15. Procédé selon l'une des revendications 11 à 14, caractérisé en ce que l'on utilise en tant que produit de départ un composé de formule V dans laquelle $R^6$ et $R^7$ représentent des groupes alkyle en C1 ou C2, en particulier des groupes méthyle.

16. Procédé pour combattre les mauvaises herbes, caractérisé en ce que l'on traite les mauvaises herbes par un produit selon la revendication 9 ou 10.

17. Utilisation des composés mentionnés dans les revendications 1 à 8 en tant qu'herbicides.

**Revendications pour l'Etat contractant: AT**

1. Produit herbicide caractérisé en ce qu'il contient un composé générale

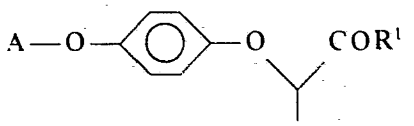

(I)

dans laquelle A représente l'un des groupes

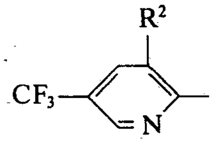

et

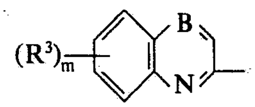

(Ia) (Ib)

dans lesquels $R^2$ représente l'hydrogène, un halogène ou un groupe trifluorométhyle et $R^3$ représente l'hydrogène, un halogène ou un groupe trifluorométhyle, m est égal à 1 ou 2, B représente —CH= ou l'azote, et $R^1$ représente le groupe

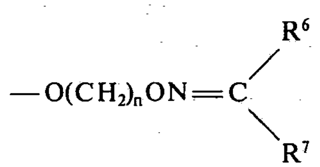

(Id)

dans lequel $R^6$ et $R^7$ représentent des groupes alkyle en C1—C6, cycloalkyle en C3 et C6, (alkyle en C1—C6)-carbonyloxy-alkyle en C1—C6, alkoxy en C1—C6, alkylthio en C1—C6 ou bien forment ensemble et avec l'atome de carbone auquel ils sont reliés, un groupe cycloalkyle contenant 4 à 7 atomes des carbone, n est égal à 1 ou 2 et $R^6$ peut également représenter l'hydrogène, et un support inerte.

2. Produit herbicide selon la revendication 1 caractérisé en ce qu'il contient un composé de formule I dans laquelle $R^2$ représente un halogène ou un groupe trifluorométhyle.

3. Produit herbicide selon la revendication 1 ou 2, caractérisé en ce qu'il contient un composé de formule I dans laquelle A représente le groupe Ib dans lequel B représente l'azote.

4. Produit herbicide selon l'une des revendications 1 à 3, caractérisé en ce qu'il contient un composé de formule I dans laquelle les substituants $R^6$ et $R^7$ sont des groupes alkyle en C1 ou C2, en particulier des groupes méthyle.

5. Produit herbicide selon l'une des revendications 1 à 4, caractérisé en ce qu'il contient l'isomère D d'un composé de formule I.

6. Produit herbicide selon l'une des revendications 1 à 5, caractérisé en ce qu'il contient du 2-[p-[(6-fluoro-2-quinoléyl)-oxy]phénoxy]-propionate d'[(isopropylidènamino)-oxy]-méthyle ou du 2-[p-[(6-chloro-2-quinoxalinyl)-oxy]-phénoxy]-propionate d'[(isopropylidènamino)-oxy]-méthyle, en particulier l'isomère D de l'un de ces composés.

7. Procédé de préparation des composés de formule générale I de la revendication 1, caractérisé en ce que l'on fait réagir un composé de formule générale

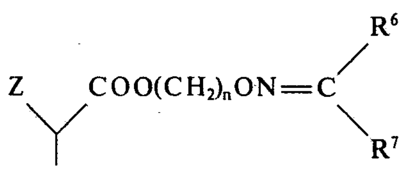

(V)

dans laquelle Z représente un groupe éliminable et $R^6$ et $R^7$ ont les significations indiquées ci-dessus, avec un composé de formule générale

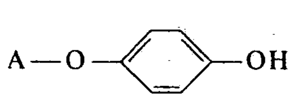

(VI)

das laquelle A a la signification indiquée ci-dessies ou un sel de métal alcalin d'un tel composé, en présence d'une base lorsque c'est nécessaire.

8. Procédé selon la revendication 7, caractérisé en ce que l'on utilise un composé de formule V dans laquelle Z représente le chlore, le brome, l'iode, un groupe méthane-sulfonyloxy ou toluène-sulfonyloxy.

9. Procédé selon la revendication 7 ou 8, caractérisé en ce que l'on utilise en tant que produits de départ de formule V les isomères D ou en ce que l'on isole l'isomère D d'un racémate obtenu.

10. Procédé selon la revendication 7, 8 ou 9, caractérisé ce que l'on utilise en tant que produit de départ de formule VI un composé dans lequel A représente le groupe Ib dans lequel B représente l'azote.

11. Procédé selon l'une des revendications 7 à 10, caractérisé en ce que l'on utilise en tant que produit de départ un composé de formule V dans laquelle $R^6$ et $R^7$ représentent des groupes alkyle en C1 ou C2, en particulier des groupes méthyle.

12. Procédé de préparation de produits herbicides, caractérisé en ce que l'on met un des composés mentionnés dans les revendications 1 à 6, par utilisation de supports solides ou liquides et le cas échéant d'autres produits auxiliaires, sous une forme appropriée à seon application.

13. Procédé pour combattre les mauvaises herbes, caractérisé en ce que l'on traite les mauvaises herbes par un produit selon l'une des revendications 1 à 6.

14. Utilisation des composés mentionnés dans les revendications 1 à 6 en tant qu'herbicides.